# EUROPEAN PATENT SPECIFICATION

(11) **EP 0 812 193 B1**
(45) Date of publication and mention of the grant of the patent: **16.10.2002**
(21) Application number: 96905772.8
(22) Date of filing: 19.02.1996
(51) Int. Cl.: A61K 31/4427, A61K 7/16

(54) **PHARMACEUTICAL COMPOSITIONS CONTAINING A BENZYDAMINE OR ITS SALT AND AN ANTIMICROBIAL AGENT**
PHARMAZEUTISCHE ZUSAMMENSETZUNGEN, WELCHE EIN BENZYDAMIN ODER DEREN SALZ UND EINEN ANTIMIKROBIELLEN WIRKSTOFF ENTHALTEN
COMPOSITIONS PHARMACEUTIQUES CONTENANT UNE BENZYDAMINE OU SON SEL ET UN AGENT ANTIMICROBIEN

(30) Priority: 28.02.1995 IT MI950379
(43) Date of publication of application: 17.12.1997
(73) Proprietor: GlaxoSmithKline S.p.A., 37135 Verona (IT)
(72) Inventor: OLDANI, Diego, I-37135 Verona (IT); FRANCESE, Franco, c/o, I-37135 Verona (IT)
(74) Representative: Reeves, Julie Frances
(86) International application number: EP9600713
(87) International publication number: WO96026724

(56) References cited:
- WO-A-94/12150
- REVUE D'ODONTOSTOMATOLOGIE DU MIDI DE LA FRANCE, vol. 29, no. 3, 1971, pages 215-222, XP000577941 BARDIER ET AL: "HEXO-IMOTRYL EN ODONTO-STOMATOLOGIE"
- REVISTA BRASILEIRA DE CLINICA E TERAPEUTICA, vol. 3, no. 3, 1974, pages 113-116, XP000577809 COSTA, HELFFENSTEIN: "THERAPEUTIC TRIAL WITH A NEW DRUG FOR THE TREATMENT OF INFLAMMATORY-INFECTIOUS DISEASES IN OTORRHINOLARINGOLOGY"
- PATENT ABSTRACTS OF JAPAN vol. 014, no. 459 (C-0767), 4 October 1990 & JP,A,02 188521 (SUNSTAR INC), 24 July 1990, & DATABASE WPI Week 9035 Derwent Publications Ltd., London, GB; AN 90-266185 & JP,A,02 188 521 (SUNSTAR KK) , 24 July 1990

## Description

This invention relates to pharmaceutical compositions and oral hygiene compositions containing an anti-inflammatory agent and a anti-microbial agent, and having improved anti-microbial activity.

Benzydamine is known as a non-steroidal anti-inflammatory agent. The preparation of benzydamine is described in US Patent No.3318905. There have been proposals to use benzydamine in mouth washes, for example in Current Therapeutical Research, Clin.Exp.,1978,23/6, (734-745).

Pathogenic oral bacteria are implicated in a number of conditions of the oral cavity, including plaque, gingivitis and periodontal disease. In addition, pathogenic fungi such as *Candida* may also be present in the oral cavity and other body cavities, and give rise to disease states, such as thrush, requiring therapy.

Agents which in the past have been suggested for use as oral antibacterial agents include cationic species such as chlorhexidine, alexidine, hexetidine and cetyl pyridinium chloride as well as non-cationic species such as triclosan. Some of these antibacterial agents are also effective as anti-fungal agents.

In many instances, a single anti-microbial agent does not have a sufficiently broad spectrum of activity to deal adequately with a wide range of pathogenic microorganisms which may be found in the oral cavity. Combining different anti-microbial agents is not always successful as the presence of one may antagonise the activity of the other.

We have now surprisingly found that benzydamine may be effectively combined with cetyl pyridinium chloride, without compromising the activity of benzydamine and with an enhancement of the activity of cetyl pyridinium chloride, so as to provide an effective antiseptic, anti-inflammatory and analgesic treatment for microbial infections especially of the gums, mouth and throat.

Accordingly, the present invention provides a pharmaceutical composition, especially an oral hygiene composition, which comprises an anti-inflammatory amount of benzydamine or a benzydamine salt, an antimicrobially effective amount of cetyl pyridinium chloride and a pharmaceutically or orally acceptable carrier or excipient.

Benzydamine may be used as a pharmaceutically acceptable salt thereof, for example benzydamine hydrochloride.

Cetyl pyridinium chloride has a combination of antibacterial and anti-fungal activity.

A composition of the invention will contain benzydamine and cetyl pyridinium chloride in the ratio (by weight) of from 10:1 to 1:10, suitably 5:1 to 1:5, and typically about 3:1 to about 1:3.

The amount of benzydamine provided in the composition of the invention is one which reaches a sufficent level on administration to have an effective and desirable anti-inflammatory effect and to enhance the activity of the anti-microbial agent. Because of the effect of the benzydamine in the composition of the invention, cetyl pyridinium chloride may be employed in an amount less than that conventionally used to establish an effective or desirable antimicrobial activity. Conventional knowledge of the use of these materials in therapy will provide a useful guide to the amounts suitable for use in the present invention, coupled with routine assays as described in the Biological Evaluation annexed to this specification.

An especially preferred composition contains benzydamine hydrochloride and cetyl pyridinium chloride in the ratio 3:1. This composition shows a synergistic effect against a wide variety of organisms such as *Propionibacterium acnes*, *Capnocytophaga gingivalis, Bacteroides oralis* and *Candida albicans*.

The composition of the invention may be formulated for topical application, for example in the form of ointments, creams, lotions, eye ointments, eye drops, ear drops, sprays, swabs, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to human patients, it is expected that the daily dosage level of the active agent will be from 0.01 to 10 mg/kg, typically around 1 mg/kg. The physician in any event will determine the actual dosage and interval of dosing that will be most suitable for an individual patient and will vary with the age, weight and response of the particular patient. The above dosages are exemplary of the average case. There can, of course, be individual instances where higher or lower dosage ranges are merited, and such are within the scope of this invention.

With the indicated dose range, no adverse toxicological effects will be observed with the compounds of the invention which would preclude their administration to suitable patients.

For use in oral hygiene, compositions of the present invention maybe provided in any of the presentations normally used for such products, for instance, dentifrices including toothpastes and toothpowders, abrasive and non-abrasive gels, mouthwashes, gargles, irrigating solutions, mouthsprays and presentations for sucking or chewing by the user such as gums, pastilles and lozenges. Components for the orally acceptable carrier or excipient will be selected according to the particular type of presentation involved.

Such compositions will contain appropriate formulating agents such as abrasives, surfactants, humectants, thickening agents, flavouring agents, sweetening agents, opacifying agents, preservatives and water, selected from those conventionally used in the oral hygiene composition art for such purposes and which are compatible with benzydamine and cetyl pyridinium chloride.

Suitable surfactants for use in compositions according to the present invention include, for instance, anionic, nonionic, cationic and amphoteric surfactants or mixtures thereof. It will be appreciated by those skilled in the art that in order to ensure that the efficacy of cetyl pyridinium chloride is not substantially diminished, compatible components will be selected for inclusion in the orally acceptable carrier or excipient. Accordingly, the skilled man will readily appreciate that where necessary anionic species may need to be avoided as such species may cause inactivation of cetyl pyridinium chloride by the formation of an insoluble precipitate therewith. Thus, anionic surfactants and anionic thickening agents are often preferably to be rejected in favour of non-anionic counterparts such as nonionic, cationic or amphoteric surfactants and nonionic thickening agents.

Where usable, suitable anionic surfactants include alkali metal (C₁₂₋₁₈)alkyl sulphates, for instance sodium lauryl sulphate, and N-acyl sarcosinates and N-acyl taurines in which the acyl moiety has from 12 to 16 carbon atoms, for instance, N-lauroyl, N-myristoyl and N-palmitoyl sarcosine alkali metal salts.

Suitable nonionic surfactants include, for example, polyethoxylated sorbitol esters, in particular polyethoxylated sorbitol monoesters, for instance, PEG(40) sorbitan di-*iso*stearate, and the products marketed under the trade name 'Tween' by ICI; polycondensates of ethylene oxide and propylene oxide (poloxamers), for instance the products marketed under the trade name 'Pluronic' by BASF-Wyandotte; condensates of propylene glycol; polyethoxylated hydrogenated castor oil, for instance, cremophors; and sorbitan fatty esters.

Suitable amphoteric surfactants include, for example, long chain imidazoline derivatives such as the product marketed under the trade name 'Miranol C2M' by Miranol; long chain alkyl betaines, such as the product marketed under the tradename 'Empigen BB' by Albright + Wilson, and long chain alkyl amidoalkyl betaines, such as cocamidopropylbetaine, and mixtures thereof.

Suitable cationic surfactants include the D,L-2-pyrrolidone-5-carboxylic acid salt of ethyl-N-cocoyl-L-arginate, marketed under the trade name CAE by Ajinomoto Co. Inc., and cocamidopropyl PG dimonium chloride phosphate and lauramidopropyl PG dimonium chloride phosphate, available under the trade names Monaquat PTC and Monaquat PTL, respectively, from Mona Corporation.

Advantageously, the surfactant is present in the range 0.005 to 20%, preferably 0.1 to 10%, more preferably 0.1 to 5% by weight of the dentifrice.

Suitable thickening agents include, for instance, nonionic thickening agents such as, for example, (C₁₋₆)alkylcellulose ethers, for instance methylcellulose; hydroxy(C₁₋₆)alkylcellulose ethers, for instance hydroxyethylcellulose and hydroxypropylcellulose; (C₂-₆)alkylene oxide modified (C₁₋₆)alkylcellulose ethers, for instance hydroxypropyl methylcellulose; and mixtures thereof. Other thickening agents such as natural and synthetic gums or gum like material such as Irish Moss, gum tragacanth, sodium carboxymethylcellulose, polyvinyl pyrrolidone, starch and thickening silicas may also be used.

Advantageously the thickening agent is present in the range 0.01 to 30%, preferably 0.1 to 15%, more preferably 1 to 5%, by weight of the composition.

Suitable humectants for use in compositions of the invention include for instance, glycerine, sorbitol, propylene glycol or polyethylene glycol, or mixtures thereof; which humectant may be present in the range from 5 to 70%, preferably 5 to 30%, more preferably 10 to 30% by weight of the composition.

Suitable abrasives for use in dentifrice compositions of the present invention include calcium carbonate, calcium phosphates, calcium pyrophosphate, insoluble sodium metaphosphate, sodium aluminosilicate, alumina, hydrated alumina, zinc orthophosphate, plastic particles, and silica, of which silica is the preferred abrasive.

Suitable silicas include natural amorphous silicas, such as, for instance, diatomaceous earth, and synthetic amorphous silicas, such as precipitated silicas and silica gels, including silica xerogels. Suitable silica xerogels are described in US 3,538,230. Suitable grades of precipitated silicas have BET surface areas in the range 20 to 300, preferably 20 to 100 m²/g and median agglomerate sizes in the range 2 to 50, preferably 5 to 30m.

Suitable precipitated silicas and silica xerogels are those marketed under the trade names Sident and Syloblanc, by Degussa and W R Grace Corporation Davison Chemical Division, respectively.

Advantageously, the silica is a "low anion" silica. As used herein, the term "low-anion" silicas refers to those in which anionic impurities such as sodium sulphate and sodium silicate which normally arise during the course of the manufacturing process are kept to a minium, through careful control of the manufacturing process. "Low anion" silicas suitably have less than 1%, preferably less than 0.5% advantageously less than 0.25% by weight of anionic impurities.

Suitable such "low anion" silicas are described in EP 0 368 130 (Proctor & Gamble), EP 0 315 503 and EP 0 396 459 (Rhone-Poulenc) and WO 90/05113 (J.M. Huber Corp). Alternatively, grades of commercially available silica with ionic impurities may be rendered suitable by washing thereof with deionised water. Conductivity measurements on the water after washing may be used to monitor the efficacy of such washing. Suitably the conductivity of the water after washing is reduced to less than 200µSiemens/cm. Suitable "low anion" silicas include the grade RP93 available from Rhone-Poulenc.

Suitably, compositions will have from 5 to 80%, preferably from 10 to 60% by weight of the abrasive.

Suitable mouthwash formulations will have an aqueous base comprising water or aqueous ethanol, and optionally a further liquid such as glycerin or propylene glycol. A surfactant may also be included, to improve the sensory properties of the composition. Mouthwash compositions may be provided in a "ready to use" form; as a concentrated solution, for dilution by the user immediately prior to use; or in solid form, such as a tablet or in a sachet, for dissolution by the user immediately prior to use. Tablets may suitably be prepared using xylitol and/or sorbitol as the major ingredient. The sachets and tablets may be formulated to provide, on dissolution, a still mouthwash, or, by the incorporation of a suitable effervescent couple, for instance sodium carbonate/bicarbonate and citric acid, an effervescent mouthwash.

Oral hygiene compositions of the present invention may usefully further comprise an anti-caries agent, for instance a source of fluoride ions such as an alkali metal or amine fluoride salt, for example sodium fluoride, tin (II) fluoride. Alternatively, the fluoride ion source may be an alkali metal monofluorophosphate salt, for example sodium monofluorophosphate, optionally used in combination with an agent such as calcium glycerophosphate which is known to enhance the activity of monofluorophosphate (GB 1 384 375, Beecham Group). Suitably the composition will comprise between 250 and 2500ppm, preferably 500 and 1500ppm of fluoride ions.

Oral hygiene compositions of the present invention may also comprise other active agents conventionally used in oral hygiene compositions, for instance:
an anti-plaque agent other than those already mentioned; an anti-calculus agent such as a tetra- or a di-alkali metal pyrophosphate salt, or a mixture thereof, an alkali metal tripolyphosphate salt or an azacycloheptane diphosphonate salt; or
an anti-sensitivity agent such as strontium acetate, strontium chloride or a potassium salt such as potassium nitrate, potassium chloride or potassium citrate.
Such agents will be included at levels to provide the desired therapeutic effect.

Oral hygiene compositions according to the present invention will have a pH which is orally acceptable, typically ranging from about pH 4 to 10.

Oral hygiene compositions according to the present invention may be prepared by admixing the ingredients in the appropriate relative amounts in any order that is convenient and thereafter and if necessary adjusting the pH to give the final desired value.

Compositions of this invention may be used in therapy or as a prophylatic against microbial infections accompanied by irritation, such as vaginal and skin infections. In particular, they may be used for oral care: as an antiseptic, anti-inflammatory and analgesic treatment of gums, mouth and throat, especially for treatment of plaque, gingivitis and periodontitis. They are useful for treating sore or bacteria infected throat, cough-irritated sore throat, and other oral ailments wherein the source of irritation is partly or wholly derived from microbial infection. They may be used for painful inflammatory conditions of the mouth and throat including pharyngitis (eg following tonsillectomy), aphthous ulcers, aspecific odontostomatoligic affections, glossitis and stomatitis and may be used before and/or after dental extractions.

The invention is further illustrated by the following Examples.

### Example 1

A mouth wash was made up as follows:

| | |
|---|---|
| Cetyl pyridinium chloride | 0.050 gm |
| Benzydamine hydrochloride | 0.150 gm |
| Glycerol | 6.000 gm |
| Ethanol | 5.000 gm |
| Sodium saccharin | 0.075 gm |
| Ethoxylated hydrogenated castor oil | 0.250 gm |
| Blue colouring | 0.001 gm |
| Yellow colouring | 0.002 gm |
| Mint flavour | 0.100 gm |
| Distilled water | to 100 ml |

### Example 2

Analgesic lozenges were made from the following components, (per lozenge):

| | |
|---|---|
| Cetyl pyridinium chloride | 1.0 mg |
| Benzydamine hydrochloride | 3.0 mg |
| Sorbitol | 1138.0 mg |
| Mannitol | 300.0 mg |
| Magnesium stearate | 40.0 mg |
| Mint flavour | 10.0 mg |
| Aspartame | 8.0 mg |

### Example 3

An oral spray was made up as follows:

| | |
|---|---|
| Cetyl pyridinium chloride | 0.500 gm |
| Benzydamine hydrochloride | 0.150 gm |
| Glycerol | 10.000 gm |
| Ethanol | 30.000 gm |
| Sodium saccharin | 0.100 gm |
| Ethoxylated hydrogenated castor oil | 0.250 gm |
| Mint flavour | 0.500 gm |
| Distilled water | to 100 ml |

### Biological Evaluation

The antimicrobial activity of a combination of cetyl pyridinium chloride and benzydamine hydrochloride (1:3 by weight) was compared with that of the individual components in a M.I.C. assay against a variety of organisms implicated in oral and vaginal infections.

The results were as follows (C = cetyl pyridinium chloride, B = benzydamine hydrochloride, C + B = 1:3 by weight mixture).

| Microorganism | | | MIC (mcg/ml) |
|---|---|---|---|
| | C (°) | B (°) | C + B (°) |
| Peptostreptococcus anaerobius | 3.9 | 750 | <0.2 + 0.7 |
| Peptostreptococcus magnus | 7.8 | 750 | 0.9 + 2.9 |
| Veillonella parvula | 7.8 | 375 | 1.9 + 5.9 |
| Veillonella parvula | 3.9 | 375 | <0.2 + 0.7 |
| Veillonella alcalescens | 7.8 | 375 | 1.9 + 5.9 |
| Veillonella spp | 7.8 | 375 | 1.9 + 5.9 |
| Lactobacillus acidophilus | 7.8 | 750 | 0.2 + 0.7 |
| Lactobacillus acidophilus | 3.9 | 750 | <0.2 + 0.7 |
| Lactobacillus acidophilus | 3.9 | 750 | <0.2 + 0.7 |
| Lactobacillus casei | 7.8 | 750 | <0.2 + 0.7 |
| Actinomyces naeslundi . | 3.9 | 375 | <0.2 + 0.7 |
| Actinomyces naeslundi | 7.8 | 750 | 0.2 + 0.7 |
| Actinomyces viscosus | 3.9 | 750 | 0.2 + 0.7 |
| Actinomyces viscosus | 7.8 | 375 | 0.2 + 0.7 |
| Actinomyces odontolyticus | 3.9 | 375 | <0.2 + 0.7 |
| Propionibacterium acnes | 3.9 | 375 | <0.2 + 0.7 |
| Capnocytophaga gingivalis | 7.8 | 375 | 1.9 + 5.9 |
| Capnocytophaga ochracea | 3.9 | 750 | 1.9 + 5.9 |
| Bacteroides fragilis | 3.9 | 375 | <0.2 + 0.7 |
| Bacteroides fragilis | 3.9 | 375 | <0.2 + 0.7 |
| Bacteroides oralis | 7.8 | 750 | <0.2 + 0.7 |
| Bacteroides oralis | 7.8 | 750 | <0.2 + 0.7 |
| | | | |
| C.albicans 1 | 3.9 | 750 | 3.9 + 11.7 |
| 2 | 3.9 | 187 | 1.9 + 5.8 |

## Claims

1. A pharmaceutical composition which comprises an anti-inflammatory amount of benzydamine or a benzydamine salt, an antimicrobially effective amount of cetyl pyridinium chloride and an pharmaceutically acceptable carrier or excipient.

2. A pharmaceutical composition according to claim 1, formulated for topical application.

3. An oral hygiene composition, which comprises an anti-inflammatory amount of benzydamine or a benzydamine salt, an antimicrobially effective amount of cetyl pyridinium chloride and an orally acceptable carrier or excipient.

4. A composition according to any one of claims 1 to 3, in which the benzydamine salt is benzydamine hydrochloride.

5. A composition according to any one of claims 1 to 4, in which the ratio of benzydamine or benzydamine salt to cetyl pyridinium chloride is from 1:3 to 3:1 by weight.

## Patentansprüche

1. Pharmazeutische Zusammenseztung, umfassend eine entzündungshemmende Menge an Benzydamin oder einem Benzydaminsalz, eine antimikrobiell wirksame Menge an Cetylpyridiniumchlorid und einen pharmazeutisch akzeptablen Träger oder Exzipienten.

2. Pharmazeutische Zusammensetzung nach Anspruch 1, für die topische Anwendung formuliert.

3. Orale Hygienezusammensetzung, umfassend eine entzündungshemmende Menge an Benzydamin oder einem Benzydaminsalz, eine antimikrobiell wirksame Menge an Cetylpyridiniumchlorid und einen oral akzeptablen Träger oder Exzipienten.

4. Zusammensetzung nach einem der Ansprüche 1 bis 3, worin das Benzydaminsalz Benzydaminhydrochlorid ist.

5. Zusammensetzung nach einem der Ansprüche 1 bis 4, worin das Verhältnis von Benzydamin oder Benzydaminsalz zu Cetylpyridiniumchlorid von 1:3 bis 3:1, bezogen auf das Gewicht, ist.

## Revendications

1. Composition pharmaceutique qui comprend une quantité anti-inflammatoire de benzydamine ou d'un sel de benzydamine, une quantité efficace du point de vue antimicrobien, de chlorure de cétylpyridinium et un véhicule ou excipient pharmaceutiquement acceptable.

2. Composition pharmaceutique selon la revendication 1, formulée en vue de l'application topique.

3. Composition d'hygiène orale qui comprend une quantité anti-inflammatoire de benzydamine ou d'un sel de benzydamine, une quantité efficace du point de vue antimicrobien, de chlorure de cétylpyridinium et un véhicule ou excipient oralement acceptable.

4. Composition selon l'une quelconque des revendications 1 à 3, dans laquelle le sel de benzydamine est le chlorhydrate de benzydamine.

5. Composition selon l'une quelconque des revendications 1 à 4, dans laquelle le rapport pondéral (benzydamine ou sel de benzydamine)/(chlorure de cétylpyridinium) est compris entre 1/3 et 3/1.
